# EUROPEAN PATENT SPECIFICATION

(11) **EP 1 802 975 B1**
(45) Date of publication and mention of the grant of the patent: **16.05.2012**
(21) Application number: 05796936.2
(22) Date of filing: 13.10.2005
(51) Int. Cl.: G01N 33/566, G01N 33/68

(54) **METHOD FOR IDENTIFYING COMPOUNDS THAT AFFECT A TRANSPORT OF A PROTEIN THROUGH A MEMBRANE TRAFFICKING PATHWAY**
VERFAHREN ZUR IDENTIFIZIERUNG VON VERBINDUNGEN MIT WIRKUNG AUF EINEN TRANSPORT EINES PROTEINS DURCH EINEN MEMBRAN-TRAFFICKING-WEG
PROCEDE POUR L'IDENTIFICATION DE COMPOSES QUI AFFECTENT LE TRANSPORT D'UNE PROTEINE A TRAVERS LA VOIE DE TRAFIC MEMBRANAIRE

(30) Priority: 13.10.2004 EP 04024393
(43) Date of publication of application: 04.07.2007
(73) Proprietor: Evotec Technologies GmbH, 40225 Düsseldorf (DE); RWTH Aachen, 52062 Aachen (DE)
(72) Inventor: FISCHER, Rainer, 52156 Monschau (DE); EMANS, Neil, B-4890 Thimister-Clermont (BE); DI FIORE, Stefano, 41462 Neuss (DE); JOCHEMS, Carlo, D-51105 Köln (DE); HERRENKNECHT, Kurt, 21077 Hamburg (DE); HURLING, Stephan, 22589 Hamburg (DE)
(74) Representative: von Kreisler Selting Werner
(86) International application number: PCT/EP2005/055220
(87) International publication number: WO 2006/040337

(56) References cited:
- WO-A-03/002977
- WO-A-2004/010145
- US-A1- 2004 033 517
- ARUN K H S ET AL: "Green fluorescent proteins in receptor research: An emerging tool for drug discovery" JOURNAL OF PHARMACOLOGICAL AND TOXICOLOGICAL METHODS, ELSEVIER, NEW YORK, NY, US, vol. 51, no. 1, January 2005 (2005-01), pages 1-23, XP004676474 ISSN: 1056-8719
- GABORIK Z ET AL: "Intracellular trafficking of hormone receptors" TRENDS IN ENDOCRINOLOGY AND METABOLISM, ELSEVIER SCIENCE PUBLISHING, NEW YORK, NY, US, vol. 15, no. 6, 1 August 2004 (2004-08-01), pages 286-293, XP004551375 ISSN: 1043-2760
- FOSTER N ET AL: "Lysosomal traffic of liganded endothelin B receptor" BIOCHIMICA ET BIOPHYSICA ACTA. MOLECULAR CELL RESEARCH, ELSEVIER SCIENCE PUBLISHERS, AMSTERDAM, NL, vol. 1642, no. 1-2, 23 September 2003 (2003-09-23), pages 45-52, XP004455357 ISSN: 0167-4889
- MARCHESE A ET AL: "The ins and outs of G protein-coupled receptor trafficking" TIBS TRENDS IN BIOCHEMICAL SCIENCES, ELSEVIER PUBLICATION, CAMBRIDGE, EN, vol. 28, no. 7, July 2003 (2003-07), pages 369-376, XP004440369 ISSN: 0968-0004
- PAASCHE ET AL: "Mechanisms of endothelin receptor subtype-specific targeting to distinct intracellular trafficking pathways" JOURNAL OF BIOLOGICAL CHEMISTRY, vol. 276, no. 36, 7 September 2001 (2001-09-07), pages 34041-34050, XP002322178 USA
- OH ET AL: "Role of endothelin receptor, G proteins and tyrosine kinases in caveolae budding and endocytosis" MOLECULAR BIOLOGY OF THE GENE, vol. 11, no. supplement, December 2000 (2000-12), page 323A, XP008044757

## Description

The technical problem underlying the present invention is to find a novel approach to identify compounds that affect the membrane trafficking of a protein of interest and not the housekeeping vesicle transport machinery that typically takes place at the same time.

US 2004/033517 A1 discloses compositions and methods based on the characterization of endothelial cell protein C receptor (EPCR) dependent signaling by activated protein C (APC) which acts through protease activated receptor 1 (PAR1).

WO 2004/010145 A discloses a method for identifying test substances that selectively inhibit or activate only a restricted number of transducting pathway of a G-protein-coupled receptor.

WO 03/002977 A discloses a method and system for identifying and evaluating the physiological response of an organism to a condition, such as a disease or other pathological condition, a drug or agent, an environmental condition and the like, by evaluating the expression of one or more GPCR pathway biomolecules in tissue microarrays from a plurality of patients.

Arun, K. H. S. et al., Journal of Pharmacological and Toxicological Methods, vol. 51, no. 1, pages 1-23, disclose green fluorescent proteins in receptor research.

Gaborik et al., Trends in Endocrinology and Metabolism, vol. 15, no. 6, pages 286-293 disclose intracellular trafficking of hormone receptors via vesicular uptake mechanisms.

Foster et al., Biochimica and Biophysica Acta, vol. 1642, no. 1-2, pages 45-52, disclose lyosomal traffic of liganded endothelin B receptor.

Marchese et al., Trends in Biochemical Sciences, vol. 28, no. 7, pages 369-376, disclose the ins and outs of G protein-coupled receptor trafficking.

Paasche et al., Journal of Biological Chemistry, vol. 276, no. 36, 34041-34050, disclose mechanisms of endothelin receptor subtype-specific targeting to distinct Intracellular trafficking pathways.

Oh et al., Molecular Biology of the gene, vol. 11, no. supplements, page 323a, disclose the role of endotelin receptor, G proteins and thyrosine kinases in caveolae budding and endocytosis.

The problem stated above is solved by the present invention which discloses in its most general sense a method for identifying compounds that affect a transport of a protein of interest through a specific membrane trafficking pathway according to claims 1 to 21.

The method according to the present invention is particularly suited to be applied in a scenario in which the generic membrane trafficking pathway and the specific membrane trafficking pathway are partially overlapping. Such an overlap may for instance occur if an enzyme is part of both such pathways.

The underlying principle of the present invention is the identification of a compound as specifically affecting the specific membrane trafficking pathway if it does not substantially affect the transport of the second marker, which may in particular be a fluorescently-labelled second marker. A second marker is typically a molecule known to be up-taken by the cell via fluid-phase endocytosis or pinocytosis.

The transport of the G-protein coupled receptor and/or the extent of endocytosis activity can be quantified using the method according to the invention.

It can be preferred, that steps a) and b) are performed simultaneously, preferably utilizing the same cells in steps a) and b). However, steps a) and b) may also be performed sequentially or conversely. One or more cell samples may be analysed at the same time.

It can be preferred, that the G-protein coupled receptor is over-expressed in the cells. Wild-type cells may also be used in the method according to the invention.

According to the invention, the G-protein coupled receptor is expressed as a labelled protein. Especially preferred is a fluorescently-labelled protein, for example a GFP-labelled protein. Generally, a luminescent labelling creating a fluorescent or phosphorescent protein is suitable for the method according to the invention. Other methods of labelling the G-protein coupled receptor include e.g. affinity tagging.

A second marker marker, preferably a fluorescently labelled marker, is added to the cells and its transport is studied to monitor endocytosis. Preferred soluble markers according to the invention are selected from the group comprising fluorescent dextran, proteins or other molecules known to be up-taken/transported by the cell through endocytosis.

The fluorescently-labelled G-protein coupled receptor and the fluorescently-labelled second marker may differ in their excitation and/or emission wavelength. Such an embodiment is particularly advantageous when conducting steps a) and b) at the same time. In this embodiment, a microscope comprising e.g. two detectors or a detector with spectral resolution may be applied to monitor the emission of said protein and said marker.

It may be also be possible to utilize fluorescent labels which differ in their excitation wavelength. These may be excited by different optical sources, such as lasers of a microscope. In this embodiment, the lasers alternately excite the different fluorescent labels in rapid succession, allowing for time-multiplexed detection of the respective emission signals utilizing a common detector.

According to the method of the invention, a compound is identified as specifically affecting the specific membrane trafficking pathway of a protein of interest if it does not substantially affect the transport of the fluorescently-labelled marker.

In one embodiment, the method of the present invention further comprises the step of selecting a compound that affects the endocytosis only minimally as a starting point for its chemical modification with a view to optimizing the compound's specificity for affecting the specific membrane trafficking pathway.

Optical, biochemical or physiological techniques for steps a) and b) of the method according to the invention can be employed. Preferably, the optical technique is of spectroscopic or microscopic nature, especially confocal microscopy or multi-photon excitation microscopy.

Employing the method according to the invention allows the monitoring or measurement of the delivery of the G-protein coupled receptor to the endocytotic pathway as well as to the recycling endosome.

As already mentioned, the generic activity of the endocytosis can be monitored or measured. It is disclosed that the membrane trafficking pathway is exocytosis and neurotransmission.

It can be preferred that the compounds that are identified using the method according to the invention affect the transport of the G-protein coupled receptor at defined stages of the trafficking pathway. These compounds can be selected from the group comprising kinase inhibitors and phosphatase inhibitors. Especially preferred compounds comprise inhibitors of protein kinases C, A and G.

The protein of interest whose transport through a plasma membrane trafficking pathway is analysed using the method according to the invention is a member of the family of G-protein coupled receptors. The receptor may be a cell surface receptor which may be a determinant of the hormonal responsiveness of a tissue or organ. Especially preferred is the endothelin A receptor.

The method according to the invention can be performed using live cells as well as cells that were chemically fixated.

The method according to the invention can be used to generate a database containing information on:
a) compounds acting only on a specific membrane trafficking pathway
b) compounds acting only on endocytosis
c) compounds acting on both pathways
d) compounds acting on neither pathway

A database which is generated as explained above is also disclosed. This database may contain compounds that were found to be acting to a minor degree un-specifically on endocytosis or specific membrane trafficking pathway. Said compounds can subsequently be structurally optimized to create compounds with an improved specificity.

The method according to the invention will be exemplified using a member of the family of G-protein coupled receptors, namely the endothelin A receptor (ETAR).

G-protein coupled receptors (GPCRs) play a key role in one of the most sensitive mechanisms used by cells to sense and respond to changes in their environment. They regulate a very broad range of responses, and this is reflected in their importance as pharmaceutical targets - 50% of drugs are estimated to directly or indirectly target GPCRs. After activation by hormone/ligand binding, GPCRs expend their signalling activity at the cell surface and many are selectively sorted and internalized. Internalization is a key step in receptor resensitization but the mechanisms that regulate internalization of GPCRs are not fully understood. Ligand binding triggers the phosphorylation of the GPCR by a GPCR kinase and initiates the interaction with its adaptor, beta-arrestin, and hence its internalization, after which GPCRs interact with many proteins controlling their trafficking through endosomes. In drug screening, compounds modulating the activity of molecules, such as enzymes, in the pathway downstream from the GPCR are searched for. Such molecules might not only be part of the specific membrane trafficking pathway relating to the GPCR, but might also be involved in generic membrane pathways. It is therefore crucial to distinguish between compounds specific and unspecific mode of action. The present invention provides a solution to this important problem of drug discovery.

Two endothelin receptor isoforms - the type A (ETAR) and B (ETBR) receptor - have been extensively characterized. These GPCRs bind endothelins (ETs), a class of peptide hormones with strong vasoactive properties. The two receptor isoforms have different cell type distribution and physiologies. The A receptor is mainly located on vascular smooth muscle cells; on endothelin binding, it promotes long-lived vasoconstriction. The B receptor is mainly located on endothelial cells; on endothelin binding, it produces a short-lived vasodilatatory response. The B receptor shows very high affinity for ETs and very long binding half-life and, because of this, is involved in clearance of ETs from the blood stream.

The two receptors have different endocytotic trafficking pathways and target destinations. The ETAR is localized on the plasma membrane until stimulated by endothelins, such as endothelin-1 (ET-1) towards which the receptor shows the highest binding affinity. ET-1 triggers ETAR internalization into a peri-centriolar recycling endosome. In contrast, ETBR binds several ET isoforms, such as ET-1, -2 and -3, is constitutively internalized and is not recycled but transported to the lysosome for degradation.

The method according to the invention will be explained in detail in the following examples.

### Examples

### Materials and Methods

### Chemicals and reagents

All fine chemicals were purchased from Sigma-Aldrich. Fluorophores and their reactive forms were purchased from Molecular Probes (Eugene, USA) and the nuclear stain DRAQ5 was from BioStatus (Shepshed, UK). 40 kDa BODIPY-FI-dextran was synthesized from amino dextran using standard protocols. The lyophilized product had a typical labelling ratio of 3 ftuorophores·mol⁻¹. Kinase and phosphatase inhibitors were purchased as 95-99% pure 10 mM stock solutions in dimethylsulfoxide or water (Biomol Hamburg, Germany). Stock solutions and formatted assay plates were stored at -20°C.

### Cell lines and cell culture

Hela cells were obtained from the German Collection of Microorganisms and Cell Cultures (Braunschweig, Germany). Hela cells were cultivated in phenol red free Dulbecco's modified eagles medium (Invitrogen; Carlsbad, USA) supplemented with 10% foetal calf serum (Biochrom; Berlin, Germany) and 1% penicillin streptomycin (Invitrogen; Carlsbad, USA). HEK 293 cells were cultivated in Dulbecco's modified eagle's medium/F12 (Invitrogen; Carlsbad, USA) supplemented with 10% foetal calf serum, 1% penicillin streptomycin and 1% genetecin.

A human endothelin A receptor cDNA clone in pCDNA3.1(-) (Invitrogen Carlsbad, USA) was excised via *Kpn*I/*Hind*III and fused to the *egfp* coding sequence of the pEGFP-N1 vector (Clontech, Palo Alto USA) by splice overlap PCR using specific primers. The amplified fragment was digested via *Kpn*I/*Eco*RV and ligated in the pCDNA3.1(-) vector digested with the same combination of restriction enzymes. The resulting pETAR-EGFP DNA was cloned, checked by sequencing and used for transfection of HEK 293 cells using standard protocols. A recombinant clone was obtained through several cycles of genetecin selection and limiting dilutions. For screening, cells were passed onto coverslip bottomed 96 well plates (Greiner; Longwood, USA, or Whatman; Brentford, UK) at a density of 4·10³ cells/well for Hela and 2·10⁴ cells/well for HEK293 cells, 48 hours in advance.

### Cell imaging

The endothelin receptor translocation assay and the fluid phase endocytosis assay were screened on the Opera ultra-high throughput confocal screening system (Evotec Technologies, Hamburg; Germany). The Opera is a fully automated 3 colour laser excitation confocal system based on an inverted microscope architecture to image cells cultivated in coverslip bottomed microtitre plates. 488, 532 or 633 nm laser excitation is delivered to a dual wheel Nipkow spinning disk that ensures confocality. Images were acquired using an Olympus 20 x 0.7 numerical aperture water immersion objective mounted on a piezo element for focusing and fitted at the top with a sealing ring and a perfusion system to permit automated water immersion and water withdrawal at the face of the objective. Emitted light from the sample was delivered to a 630 nm dichroic mirror that split the emitted light between two 12 bit high quantum efficiency 1.3 megapixel CCD cameras. All operating parameters for the instrument were controlled by the Opera software running on a 2 Ghz PC connected to a 1 Gbps local network of three image analysis computers. Image alignment, flat field and background correction were automatically performed based on reference and orientation images. Automated experimental acquisition protocols were defined for each screening based on trial samples. 12 bit grey scale image pairs were captured, quantitatively analysed and stored in a compressed format. Images were archived within a 240 GB storage database or automatically exported as 16 bit TIFF files for analysis in Metamorph (Universal Imaging; West Chester, PA) or for presentation.

Coverslip grown cells were imaged using a Leica TCS SP confocal using 488 nm and 568 nm excitation and 510-530 and 590-620 nm emission filter settings, respectively. Images were processed and overlaid using Metamorph.

### Cell based assay screening protocols

Compounds in DMSO or H₂O were aliquoted into 96 well v-bottomed screening plates (Matrix Technologies; Hudson, USA) before screening and stored at - 20°C in the assay format. Compounds were diluted into 1% serum (HEK screens) or serum free (Hela screens) buffered media at the working concentration (0.1 nM to 10 µM) just prior to screening.

For the GPCR internalization assay, recombinant HEK cells grown were plated in 96 well plates and incubated overnight in DMEM/F12 containing 1% FBS. The plates were washed and the cells incubated for 120 min under tissue culture conditions with the compounds in 1% serum medium. The compound solutions were then exchanged for a solution with the same compound concentration and supplemented with 40 nM endothelin-1 and 10 µM Syto60. Cells were incubated for 120 min at 22°C. Plates were imaged by the Opera using 488/633 nm excitation, 3 µm focus height above the coverslip, 250 ms or 500 ms exposure and 510 nm (50 nm bandpass) or 680 (50 nm bandpass) filters, respectively. Typically, 5 image pairs (at 488 and 633 nm excitation) per well were acquired.

For the fluid phase endocytosis assay, Hela cells in 96 well plates were washed and incubated for 120 min with the compounds in serum free buffered medium under tissue culture conditions. The medium was then replaced with serum-free buffered medium supplemented with 1 mg/ml BODIPY-FL-Dextran and 10 µM Syto60. Cells were incubated for 20 min at 37°C, then placed on a cooled block and washed extensively with ice cold phosphate buffered saline 1% w/v bovine serum albumin (Serva; Heidelberg, Germany). Plates were imaged by the Opera using 488/633 nm excitation, 3 µm focus height above the coverslip, 1000 ms or 500 ms exposure and 510 nm (50 nm bandpass) or 680 (50 nm bandpass) filters, respectively.

### Cell based assay evaluation

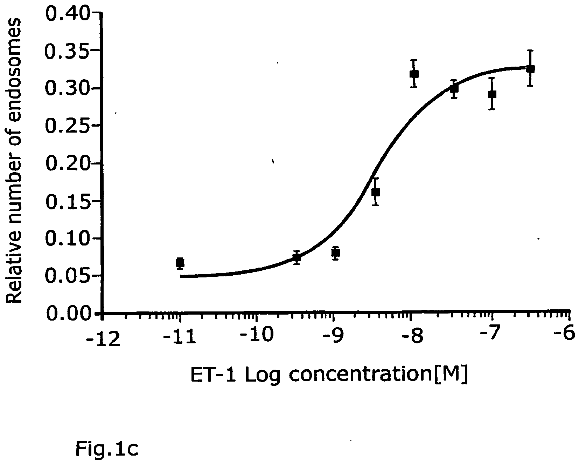

GPCR translocation was evaluated using scripts within the Acapella script player software environment of the Opera. The script measured translocation of the ETAR-EGFP fusion protein from the plasma membrane into recycling endosomes. Cells were first located in the 633 nm excitation SYTO60 image. Red fluorescent nuclei were identified by thresholding and edge seeking and then segmented to create a mask. The number of cells was counted and the nuclei mask used to generate a new cytoplasmic mask. Subsequently, each cell was individually segmented and cells with at least one bright recycling endosome were identified. Image analysis was performed on- and offline and analyses were rejected if the cell count was beneath 100 per field of view. The script passed the acceptance criteria of measuring the EC₅₀ of endothelin stimulation as 3.4 nM, with a Z' of 0.7 (Fig. 1c).

Fluid phase endocytosis was measured using custom written scripts in Metamorph. Cells were identified in the 633 nm excitation SYTO60 image, the nuclei segmented and cells counted. A mask was generated from this image and used to identify cells in overlay of the 488 nm BODIPY-FL dextran image, to create regions of interest around the cell borders and excise them from the image. Background was deducted from the processed BODIPY-FL dextran image that was thresholded to identify endosomes as individual regions of interest. Image pairs with less than 300 cells per field of view were rejected. This assay had a Z' of 0.64.

### Example 1: Measurement of GPCR internalization and fluid phase endocytosis

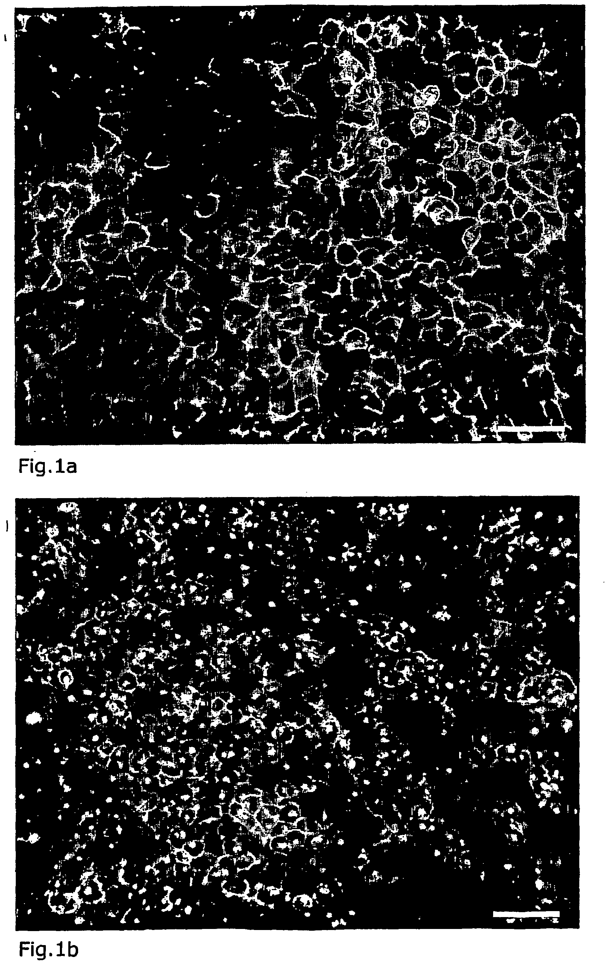

The endothelin A GPCR internalization screen was carried out using a stably transfected HEK 293 cell line expressing a fusion protein between the ETAR and a c-terminal copy of the enhanced green fluorescent protein. The ETAR fusion protein expressed at detectable levels in HEK293 cells where it localized to the plasma membrane with hardly any internal labelling (Fig. 1a). After serum deprivation for 16 hours, stimulation of stably expressing HEK293 cells with 40 nM endothelin-1 lead to internalization of the fluorescent ETAR into an intracellular peri-centriolar compartment (Fig. 1b). Co-localization experiments after red fluorescent transferrin internalization confirmed that the ETAR localized to a recycling endosome (data not shown). This internalization event was used as the basis for measuring trafficking of the receptor by image analysis. Serum deprived cells were stimulated with endothelin-1 at several concentrations and simultaneously counterstained with DRAQ5 or SYTO60, which are long wavelength excitable DNA and nucleic acid stains, respectively. The cells were stimulated for 120 min at 22-23°C, then imaged on the Opera automated confocal imaging reader. Images of the GFP and red counterstain fluorescence were automatically recorded and used to determine the number of cells where internalization of the receptor had occurred. The relative number of cells showing endosomal internalization at a range of endothelin-1 concentrations is shown in figure 1c;from these data, the EC₅₀ for endothelin was determined to be 3.4 nM. This EC₅₀ value for endothelin-1 activation of the ETAR-EGFP is in agreement with previously reported imaging and non-imaging based measurements for ETAR activation, and validates the assay as a measure of GPCR activation and internalization.

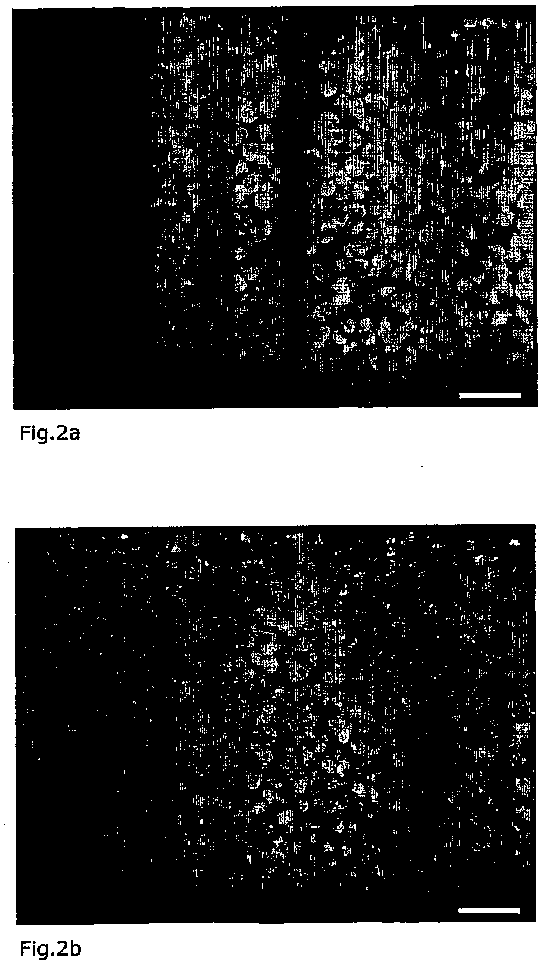

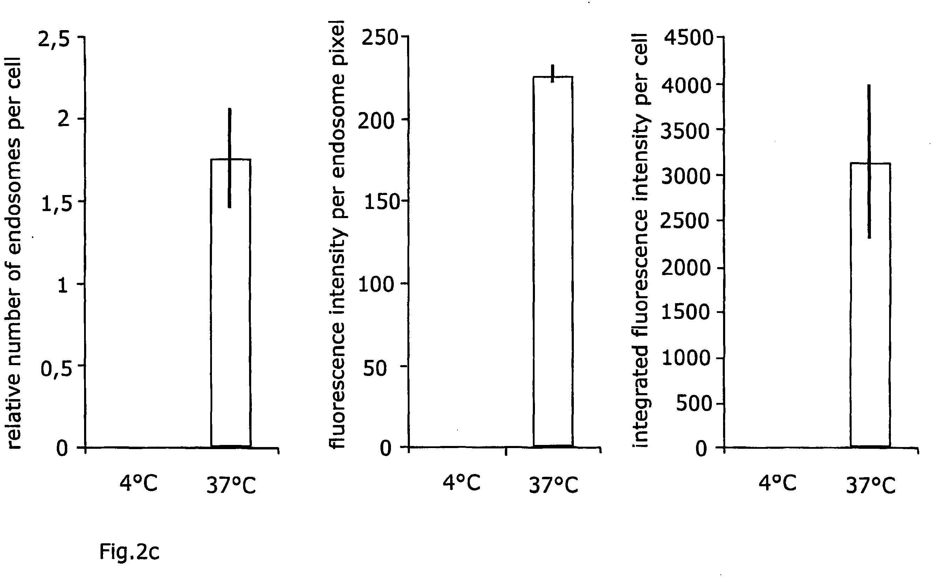

A fluid phase endocytosis assay measured the internalization of green fluorescent 40 kDa BODIPY-FI dextran. Dextrans are ideal endocytotic markers for labelling endosomes and macropinosomes. In this assay, endosomes labelled with fluorescent dextran by internalization were readily identified with the Opera instrument(Fig. 2b). Dextran internalization satisfied all the classical prerequisites for an internalization assay - it was temperature dependent, arrested at 4°C (Fig. 2a), and required a source of energy in the medium. Endocytosis was quantified by analysis of paired images of cells labelled with green dextran and the SYTO60 nuclear counter stain. The analysis was validated on cells that had internalized green fluorescent dextran for 20 min at 37°C or at 4°C (Fig. 2c). This simple one step screen of endocytosis may prove useful for genome wide screening for proteins involved in the pathway using small interfering RNAs.

### Example 2: Measuring GPCR internalization

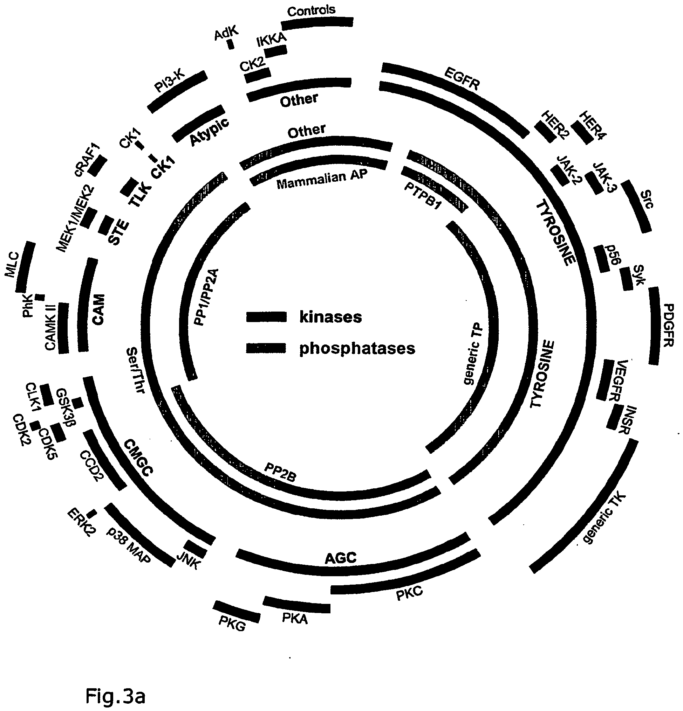

The effect of 84 kinase and phosphatase inhibitors on GPCR endocytosis was screened using the ETAR internalization assay. As shown in the rotary map in Figure 3a, the collection of compounds included inhibitors directed against kinases of eight out of nine groups/families described in the most recent classification of the human kinome and against three classes of phosphatases.

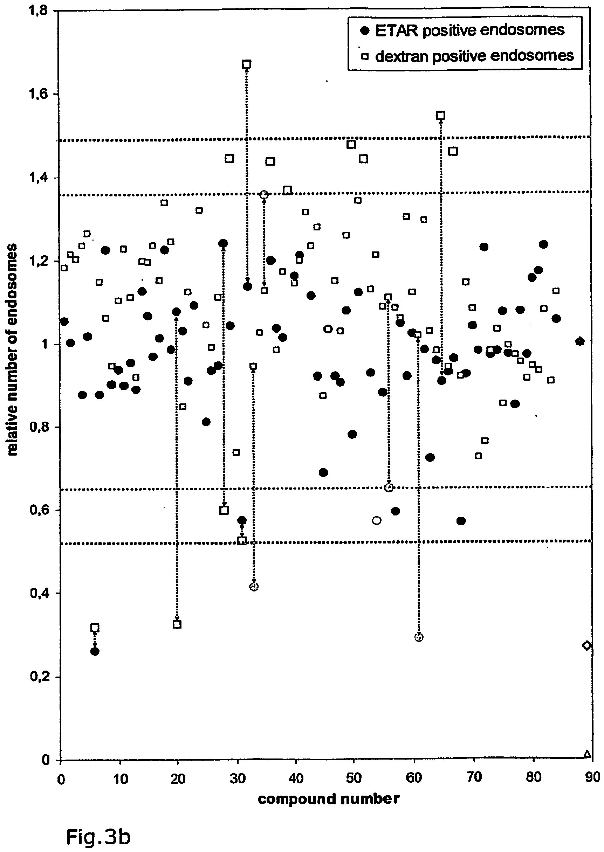

Cells were pre-treated with 10 µM compound before stimulation with 40 nM endothelin-1 to ensure a uniform and robust internalization response for the receptor. No changes in receptor distribution were detected after compound treatment alone (data not shown). Receptor internalization was measured in cells treated with the compounds by automated image analysis of 5 image pairs per compound per experiment. No internalization was observed in unstimulated or untreated cells, or in cells treated with DMSO alone (Fig. 3b). Internalization of the receptor in stimulated cells was robust, and control-stimulated and control-unstimulated measurements corresponded to the expected values from the titration curve (Fig. 1c).

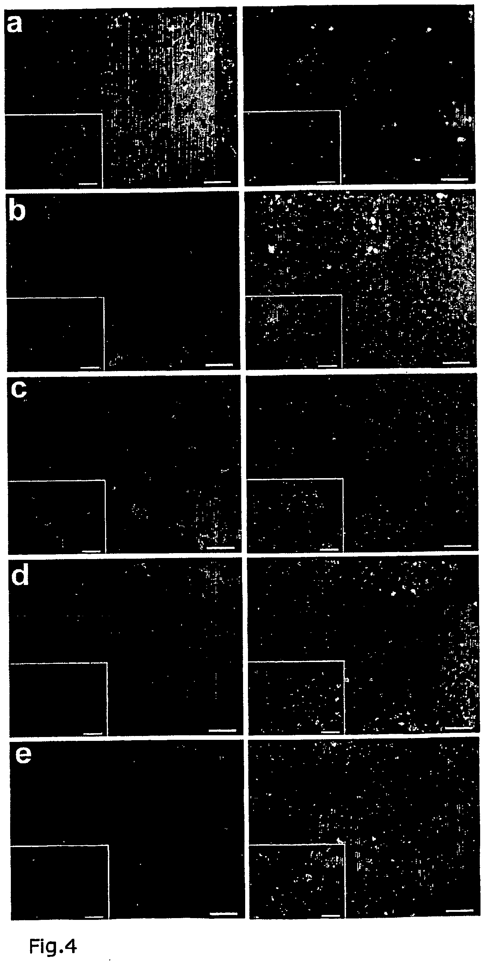

The primary screen used image analysis to identify wells where the relative amount of receptor internalization differed from the control (Fig. 3b). The output of the automated image analysis indicated that some compounds affected GPCR internalization and some had lethal effects on the cells. Primary hits were identified as those compounds that gave a response within or beyond a significance threshold set at ± two-to-three times the standard deviation of the untreated stimulated control cell population (Fig 3b). Images of the primary hit compounds were then visually screened to identify changes in receptor distribution and/or trafficking as compared to control cells. The visual screen confirmed the result of the automated analysis and further distinguished hits with actual effects on receptor trafficking from those with partial or lethal effects on the cells. A total of six compounds were selected that altered receptor trafficking. The morphologies observed with 10 µM compound comprised: arrest of the receptor at the plasma membrane (e.g. staurosporine, Ro 31-8220, Fig. 4a, and b, respectively), arrest of the receptor in small vesicular bodies, presumably early endosomal compartments (erbstatin analogue; Fig. 4c) or enhanced accumulation in the recycling endosome (H-89; Figure 4e).

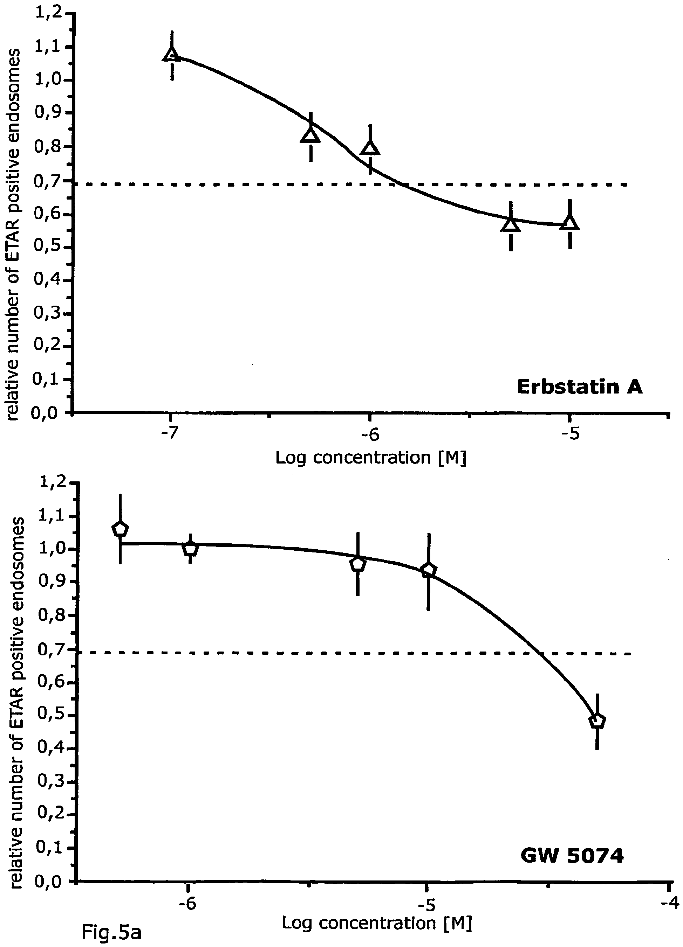

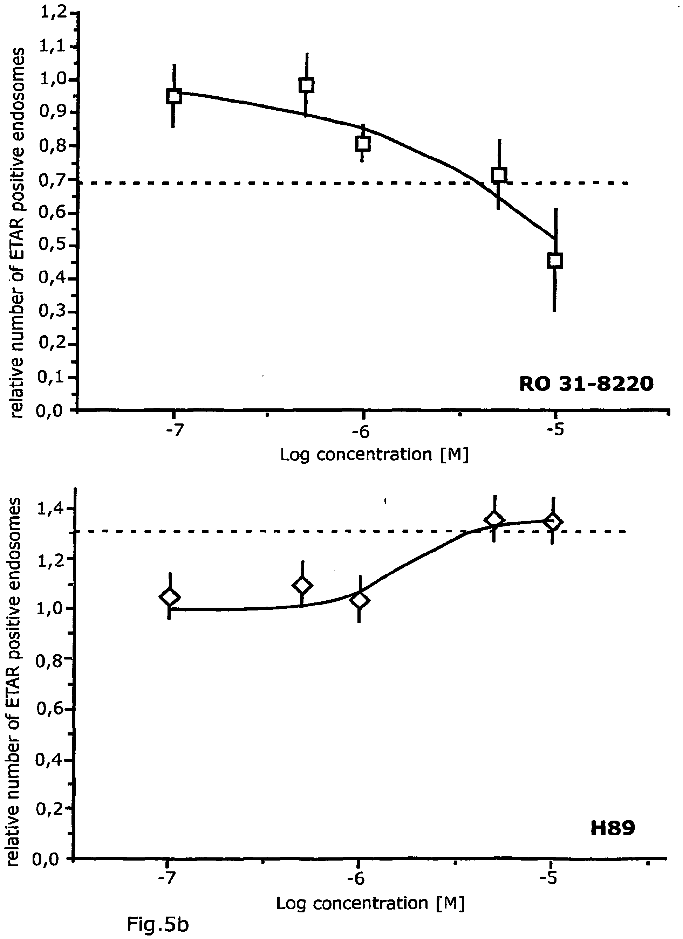

Secondary screening was performed on a subset of compounds including the hits of the primary screen (Fig. 3b). The secondary screen was performed across a range from 0.1 µM to 50 µM to assign dose dependency to the observed morphologies (Figure 5).

The most effective blocker of GPCR internalization was staurosporine, which had an effective concentration at 100 nM; in contrast GW 5074 - a partial hit in the primary screen - had effect at 50 µM (Fig. 5). GW 5074 lead to delayed accumulation of the receptor in the recycling endosomes as judged by altered shape and consistently lower fluorescence intensity of these compartments in comparison to those of untreated and endothelin-1 stimulated control cells (Fig. 4d).

### Example 3: Screening the fluid Phase endocytosis assay

To identify compounds affecting GPCR internalization and not affecting housekeeping endocytosis, the kinase and phosphatase inhibitor collection was screened against the fluid phase endocytosis assay (Fig. 3b). The effect of the compounds was evaluated. Four compounds (staurosporine, tyrphostin 9, AG-879, GF 109203X) were identified that reduced the number of endosomes (Fig. 3b) and the integrated intensity per cell relative to the control (data not shown). Of these, only staurosporine slightly increased the endosomal pixel intensity. Conversely, some compounds increased the relative number of endosomes of which two (hypericin and genistein) resulted in significantly increased integrated fluorescence intensity per cell. Visual analysis of the cells treated with the hit compounds did not identify any anomalies compared to control cells, except the absence of internalized green fluorescent dextran.

Overlap of the ETAR internalization and the fluid phase endocytosis screen was used to generate differential information from the two assays. This clearly identified molecules whose effect was congruent in both assays (e.g. staurosporine and GF 109203X; Fig. 3b) or those where there was a large difference between the responses of the two assays (e.g. Ro 31-8220, erbstatin A, H-89, GW 5074; Fig 3b).

## Claims

1. A method for identifying compounds that affect a transport of a G-protein coupled receptor of interest through a specific membrane trafficking pathway mediated by said receptor within the context of endocytosis, which is not mediated by said receptor, **characterised by** the following steps:
a) monitoring the transport of the G-protein coupled receptor of interest in a cell in the presence of the compound, wherein the G-protein coupled receptor is labelled with a first marker,
b) monitoring the transport of a second marker through endocytosis in a cell in the presence of the compound,
c) comparing the results obtained from steps a) and b) and thereby identifying a compound affecting the specific membrane trafficking pathway.

2. The method according to claim 1, wherein the transport of the G-protein coupled receptor of interest and/or the extent of the endocytosis activity is quantified.

3. The method according to claim 1 or 2, wherein the endocytosis is fluid-phase endocytosis.

4. The method according to claims 1-3, wherein steps a) and b) are performed simultaneously, preferably utilizing the same cell for conducting steps a) and b).

5. The method according to claims 1-4, wherein the G-protein coupled receptor is over-expressed in the cells.

6. The method according to claims 1-5, wherein the G-protein coupled receptor is expressed as a labelled protein, optionally a fluorescently-labelled protein.

7. The method according to claims 1-6, wherein a labelled second marker, preferably a fluorescently labelled second marker, is added to the cells and its transport is studied as a tool for monitoring endocytosis.

8. The method according to claim 6 and 7, wherein the fluorescently-labelled protein and the fluorescently-labelled second marker differ in their excitation and/or emission wavelength.

9. The method of claim 7 and 8, wherein the labelled second marker is selected from the group comprising fluorescent dextran or other molecules, optionally fluorescently-labelled molecules, known to be uptaken by fluid-phase endocytosis.

10. The method according to claims 1-9, wherein a compound is identified as specifically affecting the specific membrane trafficking pathway if it does not substantially affect the transport of the second marker, optionally a fluorescently-labelled second marker.

11. The method according to claims 1-10, wherein steps a) and b) are performed by optical, optionally spectroscopic, microscopic, biochemical or physiological methods.

12. The method according to claim 11, wherein the microscopic method is confocal microscopy or multi-photon excitation microscopy.

13. The method according to claims 1-12, wherein the receptor of interest is the endothelin A receptor.

14. The method according to claims 1-13, wherein the delivery of the receptor to an endocytotic pathway is measured.

15. The method according to claims 1-14, wherein the delivery of the receptor to a recycling endosome is measured.

16. The method according to claims 1-15, wherein the compounds affect the transport of the receptor at defined stages of the endocytosis.

17. The method according to claims 1-16, wherein the compounds are selected from the group comprising kinase inhibitors and phosphatase inhibitors.

18. The method according to claims 1-17, wherein the compounds comprise inhibitors of protein kinases C, A and G.

19. The method according to claims 1-18, wherein the cells are live cells or cells that are chemically fixated.

20. The method according to claims 1-19 for the generation of a database containing information on:
a) compounds acting only on a specific membrane trafficking pathway;
b) compounds acting only on endocytosis;
c) compounds acting on both pathways;
d) compounds acting on neither pathway.

21. The method according to claims 1-20 further comprising the step of selecting a compound that affects endocytosis only minimally as a starting point for its chemical modification with a view to optimizing the compound's specificity for affecting the specific membrane trafficking pathway.

## Patentansprüche

1. Verfahren zum Identifizieren von Verbindungen, die den Transport eines interessierenden G-Protein-gekoppelten Rezeptors durch einen speziellen Membrantransportweg, der von dem Rezeptor vermittelt wird, im Zusammenhang mit einer Endocytose, die nicht von dem Rezeptor vermittelt wird, beeinflussen, **gekennzeichnet durch** die folgenden Schritte:
a) Überwachen des Transports des interessierenden G-Protein-gekoppelten Rezeptors in einer Zelle in Gegenwart der Verbindung, wobei der G-Protein-gekoppelte Rezeptor mit einem ersten Marker markiert ist;
b) Überwachen des Transports eines zweiten Markers **durch** Endocytose in einer Zelle in Gegenwart der Verbindung;
c) Vergleichen der in den Schritten a) und b) erhaltenen Ergebnisse und **dadurch** Identifizieren einer Verbindung, die den speziellen Membrantransportweg beeinflusst.

2. Verfahren gemäß Anspruch 1, wobei der Transport des interessierenden G-Protein-gekoppelten Rezeptors und/oder das Ausmaß der Endocytoseaktivität quantifiziert werden.

3. Verfahren gemäß Anspruch 1 oder 2, wobei es sich bei der Endocytose um Flüssigphasen-Endocytose handelt.

4. Verfahren gemäß Anspruch 1 bis 3, wobei die Schritte a) und b) gleichzeitig durchgeführt werden, wobei vorzugsweise dieselbe Zelle zur Durchführung der Schritte a) und b) verwendet wird.

5. Verfahren gemäß Anspruch 1 bis 4, wobei der G-Protein-gekoppelte Rezeptor in den Zellen überexprimiert wird.

6. Verfahren gemäß Anspruch 1 bis 5, wobei der G-Protein-gekoppelte Rezeptor als markiertes Protein, gegebenenfalls als fluoreszenzmarkiertes Protein, exprimiert wird.

7. Verfahren gemäß Anspruch 1 bis 6, wobei ein markierter zweiter Marker, vorzugsweise ein fluoreszenzmarkierter zweiter Marker, zu den Zellen gegeben wird und dessen Transport als Werkzeug zur Überwachung der Endocytose untersucht wird.

8. Verfahren gemäß Anspruch 6 und 7, wobei sich das fluoreszenzmarkierte Protein und der fluoreszenzmarkierte zweite Marker in ihrer Anregungs- und/oder Emissionswellenlänge unterscheiden.

9. Verfahren gemäß Anspruch 7 und 8, wobei der markierte zweite Marker aus der Gruppe ausgewählt ist, die fluoreszierendes Dextran oder andere Moleküle, gegebenenfalls fluoreszenzmarkierte Moleküle, die bekanntermaßen durch Flüssigphasen-Endocytose aufgenommen werden, umfasst.

10. Verfahren gemäß Anspruch 1 bis 9, wobei eine Verbindung als den speziellen Membrantransportweg spezifisch beeinflussend identifiziert wird, wenn sie den Transport des zweiten Markers, gegebenenfalls eines fluoreszenzmarkierten zweiten Markers, im Wesentlichen nicht beeinflusst.

11. Verfahren gemäß Anspruch 1 bis 10, wobei die Schritte a) und b) durch optische, gegebenenfalls spektroskopische, mikroskopische, biochemische oder physiologische Verfahren durchgeführt werden.

12. Verfahren gemäß Anspruch 11, wobei es sich bei dem mikroskopischen Verfahren um konfokale Mikroskopie oder Multiphotonen-Exzitation-Mikroskopie handelt.

13. Verfahren gemäß Anspruch 1 bis 12, wobei es sich bei dem interessierenden Rezeptor um den Endothelin-A-Rezeptor handelt.

14. Verfahren gemäß Anspruch 1 bis 13, wobei die Abgabe des Rezeptors an einen Endocytoseweg gemessen wird.

15. Verfahren gemäß Anspruch 1 bis 14, wobei die Abgabe des Rezeptors an ein Recycling-Endosom gemessen wird.

16. Verfahren gemäß Anspruch 1 bis 15, wobei die Verbindungen den Transport des Rezeptors in definierten Stadien der Endocytose beeinflussen.

17. Verfahren gemäß Anspruch 1 bis 16, wobei die Verbindungen aus der Gruppe ausgewählt sind, die Kinase-Inhibitoren und Phosphatase-Inhibitoren umfasst.

18. Verfahren gemäß Anspruch 1 bis 17, wobei die Verbindungen Inhibitoren der Protein-Kinasen C, A und G umfassen.

19. Verfahren gemäß Anspruch 1 bis 18, wobei es sich bei den Zellen um lebende Zellen oder um Zellen, die chemisch fixiert sind, handelt.

20. Verfahren gemäß Anspruch 1 bis 19 zur Erzeugung einer Datenbank, die Informationen enthält über:
a) Verbindungen, die nur auf einen speziellen Membrantransportweg einwirken;
b) Verbindungen, die nur auf die Endocytose einwirken;
c) Verbindungen, die auf beide Wege einwirken;
d) Verbindungen, die auf keinen der beiden Wege einwirken.

21. Verfahren gemäß Anspruch 1 bis 20, das weiterhin den Schritt des Auswählens einer Verbindung, die die Endocytose nur minimal beeinflusst, als Ausgangspunkt für deren chemische Modifikation im Hinblick auf die Optimierung der Spezifität der Verbindung in Bezug auf die Beeinflussung des speziellen Membrantransportwegs umfasst.

## Revendications

1. Procédé d'identification de composés qui affectent un transport d'un récepteur couplé aux protéines G d'intérêt via une voie de trafic membranaire spécifique médiée par ledit récepteur dans le contexte de l'endocytose, qui n'est pas médiée par ledit récepteur, **caractérisé par** les étapes suivantes consistant à :
a) surveiller le transport du récepteur couplé aux protéines G d'intérêt dans une cellule en présence du composé, où le récepteur couplé aux protéines G est tracé avec un premier marqueur,
b) surveiller le transport d'un second marqueur par endocytose dans une cellule en présence du composé,
c) comparer les résultats obtenus aux étapes a) et b) et identifier ainsi un composé affectant la voie de trafic membranaire spécifique.

2. Procédé selon la revendication 1, dans lequel le transport du récepteur couplé aux protéines G d'intérêt et/ou l'ampleur de l'activité d'endocytose est quantifié.

3. Procédé selon la revendication 1 ou 2, dans lequel l'endocytose est une endocytose en phase fluide.

4. Procédé selon les revendications 1 à 3, dans lequel les étapes a) et b) sont réalisées simultanément, de préférence en utilisant la même cellule pour réaliser les étapes a) et b).

5. Procédé selon les revendications 1 à 4, dans lequel le récepteur couplé aux protéines G est surexprimé dans les cellules.

6. Procédé selon les revendications 1 à 5, dans lequel le récepteur couplé aux protéines G est exprimé comme une protéine tracée, facultativement une protéine tracée de façon fluorescente.

7. Procédé selon les revendications 1 à 6, dans lequel un second marqueur tracé, de préférence un second marqueur tracé de façon fluorescente, est ajouté aux cellules et son transport est étudié comme un outil pour surveiller l'endocytose.

8. Procédé selon les revendications 6 et 7, dans lequel la protéine tracée de façon fluorescente et le second marqueur tracé de façon fluorescente diffèrent dans leur excitation et/ou longueur d'onde d'émission.

9. Procédé selon les revendications 7 et 8, dans lequel le second marqueur tracé est choisi dans le groupe comprenant le dextrane fluorescent ou d'autres molécules, facultativement des molécules tracées de façon fluorescente, connues pour être captées par une endocytose en phase fluide.

10. Procédé selon les revendications 1 à 9, dans lequel un composé est identifié comme affectant spécifiquement la voie de trafic membranaire spécifique s'il n'affecte pas sensiblement le transport du second marqueur, optionnellement un second marqueur tracé de façon fluorescente.

11. Procédé selon les revendications 1 à 10, dans lequel les étapes a) et b) sont réalisées par des procédés optiques, facultativement spectroscopiques, microscopiques, biochimiques ou physiologiques.

12. Procédé selon la revendication 11, dans lequel le procédé microscopique est la microscopie confocale ou la microscopie par excitation multi-photonique.

13. Procédé selon les revendications 1 à 12, dans lequel le récepteur d'intérêt est le récepteur de l'endothéline A.

14. Procédé selon les revendications 1 à 13, dans lequel la libération du récepteur à une voie endocytotique est mesurée.

15. Procédé selon les revendications 1 à 14, dans lequel la libération du récepteur à un endosome de recyclage est mesurée.

16. Procédé selon les revendications 1 à 15, dans lequel les composés affectent le transport du récepteur à des stades définis de l'endocytose.

17. Procédé selon les revendications 1 à 16, dans lequel les composés sont choisis dans le groupe comprenant les inhibiteurs de kinase et les inhibiteurs de phosphatase.

18. Procédé selon les revendications 1 à 17, dans lequel les composés comprennent des inhibiteurs de protéine kinases de C, A et G.

19. Procédé selon les revendications 1 à 18, dans lequel les cellules sont des cellules vivantes ou des cellules qui sont chimiquement fixées.

20. Procédé selon les revendications 1 à 19, pour la génération d'une base de données contenant des informations sur :
a) des composés agissant uniquement sur une voie de trafic membranaire spécifique ;
b) des composés agissant uniquement sur l'endocytose ;
c) des composés agissant sur les deux voies ;
d) des composés n'agissant sur aucune des voies.

21. Procédé selon les revendications 1 à 20, comprenant en outre l'étape consistant à sélectionner un composé qui affecte l'endoc-ytose uniquement de façon minimale comme point de départ pour sa modification chimique avec perspective d'optimiser la spécificité du composé pour affecter la voie de trafic membranaire spécifique.
